(19) Europäisches Patentamt / European Patent Office / Office européen des brevets

(11) **EP 4 501 232 B1**

(12) **EUROPEAN PATENT SPECIFICATION**

(45) Date of publication and mention
of the grant of the patent:
**01.04.2026 Bulletin 2026/14**

(21) Application number: **23910270.0**

(22) Date of filing: **19.12.2023**

(51) International Patent Classification (IPC):
*A61B 5/276* (2021.01)    *A61B 5/25* (2021.01)
*A61B 5/316* (2021.01)    *A61B 5/318* (2021.01)
*A61B 5/369* (2021.01)    *A61B 5/389* (2021.01)

(52) Cooperative Patent Classification (CPC):
**A61B 5/053; A61B 5/25; A61B 5/30; A61B 5/305;
A61B 5/7221; A61B 5/7225; A61B 5/725;**
A61B 2560/0276

(86) International application number:
**PCT/CN2023/139896**

(87) International publication number:
**WO 2024/140340 (04.07.2024 Gazette 2024/27)**

(54) **BIOELECTRICAL SIGNAL ABNORMALITY MONITORING METHOD AND APPARATUS**

VERFAHREN UND VORRICHTUNG ZUR ÜBERWACHUNG VON ANOMALIEN BIOELEKTRISCHER SIGNALE

PROCÉDÉ ET APPAREIL DE SURVEILLANCE D'ANOMALIE DE SIGNAL BIOÉLECTRIQUE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **31.12.2022 CN 202211735686**

(43) Date of publication of application:
**05.02.2025 Bulletin 2025/06**

(73) Proprietor: **Kingfar International Inc.
100085 Beijing (CN)**

(72) Inventors:
• **ZHAO, Qichao
Beijing 100085 (CN)**
• **YANG, Ran
Beijing 100085 (CN)**

(74) Representative: **Dr. Gassner & Partner mbB
Wetterkreuz 3
91058 Erlangen (DE)**

(56) References cited:
WO-A1-2014/051590    CN-A- 102 613 969
CN-A- 102 613 969    CN-A- 116 584 950
CN-U- 205 215 225    JP-A- H11 332 839
US-A1- 2003 083 584

**Description**

TECHNICAL FIELD

The present application relates to a technical field of sensor circuit design, and, in particular, to a method and device for monitoring abnormal bioelectric signals.

BACKGROUND ART

Bioelectricity is essentially a type of physical and physical-chemical change in a process of life activities, which is a manifestation of normal physiological activities and a fundamental characteristic of living tissues. Electro X gram (EXG) includes electroencephalography (EEG), electrocardiogram (ECG), and electromyogram (EMG), collecting bioelectrograms is a detection method for studying potential and polarity changes that occur in organs, tissues, and cells during biological activities. For example, CN205215225U discloses a human body impedance measuring circuit comprising a controller, a multi-frequency signal generating circuit, a signal conversion circuit, an exciting electrode, a measuring electrode, a multi-path selection switch and a standard reference resistor; the controller controls the multi-frequency signal generating circuit to generate an alternating voltage signal, the alternating voltage signal is converted into a corresponding excitation current signal by the signal conversion circuit, the excitation current signal is input to the excitation electrode, and a voltage signal of the measuring electrode is obtained; the multi-path selection switch is controlled by the controller and selects the excitation current loop and the measurement voltage loop; in any excitation current loop, in each measuring loop, the measuring electrode measures the voltage value of the equivalent impedance of each section of the human body.

Circuit systems of bioelectrogram acquisition devices are fundamentally the same, but an accuracy of each signal is slightly different, often, there are a plurality of electrodes used for collecting bioelectrograms in the circuit, if one of the electrodes used for collecting bioelectrograms in the circuit falls off or is poorly connected, the device cannot accurately detect a potential state; even worse, when the electrode is unloaded, various electromagnetic radiation from the air can accumulate on the electrode, causing the device to detect abnormal potential waveforms. Therefore, an electrode detachment detection technology of multi-electrode bioelectrogram acquisition device, furthermore, based on electrode detachment detection technology, abnormal monitoring of bioelectric signals can be achieved, which is a very important technology.

In the existing technology for abnormal monitoring of bioelectric signals, in order to monitor connection states of each electrode, an additional bioelectric signal abnormal monitoring circuit is connected to each electrode on a basis of being connected to an acquisition circuit, then, through an optocoupler-isolation circuit, IO quantity is output to a microcontroller chip MCU to achieve active abnormal monitoring of bioelectric signals, thereby realizing abnormal monitoring of bioelectric signals.

However, actively initiating abnormal monitoring of bioelectric signals on the circuit can cause interference to the acquisition circuit, and for some devices with a large number of electrodes (such as EEG acquisition devices), each electrode lead wire needs to be connected to a bioelectric signal abnormal monitoring circuit (if there are 100 lead wires, an additional 100 monitoring circuits need to be added to a circuit board), which will increase a size of the circuit board, similarly, shell volume of the bioelectrogram acquisition device will also increase, making a product bulky.

Therefore, it is an urgent problem to provide a method for monitoring abnormal bioelectric signals in the bioelectrogram acquisition device for multi-electrode situations.

In addition, CN102613969A discloses a lead detachment detection method, comprising: collecting physiological signals from a human body and transmitting the same via lead wires; removing high-frequency noise from the physiological signals through low-pass filtering; coupling the filtered signals with a sine wave signal to generate a superimposed signal; performing impedance transformation on the superimposed signal and outputting transformed signal; differentially amplifying the transformed signal and outputting the amplified signal; converting the amplified signal from analog to digital; processing the digital signal through band-pass filtering to remove physiological signal components, thereby extracting and outputting a sine wave signal at a specified frequency; calculating the amplitude of the band-pass filtered digital signal to determine the amplitude of the sine wave signal; and comparing the obtained sine wave amplitude with a preset amplitude threshold to determine lead connection status.

JPH11332839A relates to an electrode abnormality detection circuit, comprising: a differential input amplifier circuit, wherein when the measurement electrodes detach, the differential input amplifier circuit generates a DC potential difference between the input bio-signals, amplifies this DC potential difference, and outputs the amplified signal; and a transistor circuit equipped with a transistor that operates based on an output voltage from the differential input amplifier circuit during electrode detachment and outputs an electrode abnormality detection signal.

SUMMARY

In view of this, an embodiment of the present application provides a method and device for monitoring abnormalities in biological electrical signals, aiming to eliminate or improve one or more defects present in the existing technology.

One aspect of the present application provides a method for monitoring abnormal bioelectric signals. A multi-electrode bioelectrogram acquisition device comprises a reference electrode, a right leg drive electrode, and a plurality of collection electrodes. each of the collection electrodes serves as a positive electrode of the multi-electrode bioelectrogram acquisition device, and the reference electrode serves as a negative electrode of the multi-electrode bioelectrogram acquisition device, and the method comprises the following steps:

generating a preset constant frequency noise signal, and the noise signal is a sine wave signal;

superimposing the noise signal onto the right leg drive electrode, when the right leg drive electrode does not detach, the noise signal is dissipated in human tissues and collected by the non-detached ones of the collection electrodes;

preprocessing analog signals from the reference electrode and each of the collection electrodes by a front-end circuit, and outputting the preprocessed analog signals respectively corresponding to the collection electrodes to an analog-to-digital converter, the preprocessing comprises differential amplification;

converting the preprocessed analog signals respectively corresponding to the collection electrodes into digital signals by the analog-to-digital converter, and transmitting the digital signals to an computation and analysis unit;

performing following operations by the computation and analysis unit:

performing band-stop filtering on each of the digital signals for a frequency band of the noise signal;

performing subtraction periodically on the digital signal before band-stop filtering and the digital signal after band-stop filtering are subtracted periodically at a preset time interval to obtain a difference value;

storing the difference value in a fixed length queue, and calculating a root mean square (RMS) result of data in the queue;

calculating a human impedance value based on the RMS result and a preset fitting formula; wherein, the preset fitting formula is obtained by data fitting based on the historical RMS results and historical human impedance values; and

determining a connection state of corresponding one of the collection electrodes based on the calculated human impedance value and preset impedance measurement standards, thereby realizing abnormal monitoring of bioelectric signals attributable to abnormal connection states of the collection electrodes based on periodic determining of the connection states of the collection electrodes.

In some embodiments of the present application, the noise signal is generated using self-excited oscillation of a Wen bridge or produced through a Direct Digital Frequency Synthesis DDS.

In some embodiments of the present application, the front-end circuit comprises a differential amplifier circuit, a follower circuit, and a wave filter, the analog signals from the reference electrode and each collection electrode are preprocessed through the differential amplifier circuit, the follower circuit, and the wave filter.

In some embodiments of the present application, types of the computation and analysis unit comprise a microcontroller unit MCU, a central processing unit CPU, and a personal computer PC.

In some embodiments of the present application, the determining the connection state of the corresponding one of the collection electrodes according to the calculated human impedance value and the preset impedance measurement standards, comprising: when the human impedance value does not exceed a first standard of the preset impedance measurement standards, determining that the collection electrode is in a stable connection state; when the human impedance value exceeds the first standard but does not exceed a second standard of the preset impedance measurement standards, determining that the collection electrode is in an unstable connection state; when the human impedance value exceeds the second standard, determining that the collection electrode is in a detached state.

In some embodiments of the present application, the method further comprises: generating alert information for one of the collection electrodes in a detected unstable connection state.

An other aspect of the present application provides a device for monitoring abnormal bioelectric signals, a multi-electrode bioelectrogram acquisition device comprises a reference electrode, a right leg drive electrode, and a plurality of collection electrodes, each of the collection electrodes serves as a positive electrode of the multi-electrode bioelectrogram acquisition device, and the reference electrode serves as a negative electrode of the multi-electrode bioelectrogram acquisition device, the device comprises:

a signal generator configured to generate a preset constant frequency noise signal, the noise signal is a sine wave signal;

the right leg drive electrode configured to superimpose the noise signal, when the right leg drive electrode does not detach, the noise signal is dissipated in human tissues and collected by non-detached ones of the collection electrodes;

a front-end circuit configured to preprocess analog signals from the reference electrode and each of the collection electrodes, and output the preprocessed analog signals respectively corresponding to the collection electrodes to an analog-to-digital converter, the preprocessing comprises differential amplification;

the analog-to-digital converter configured to convert the preprocessed analog signals respectively corresponding to the collection electrodes into digital signals, and transmit the digital signals respectively corresponding to the collection electrodes to an computation and analysis unit;

the computation and analysis unit configured to:

perform band-stop filtering on each of the digital signals for a frequency band of the noise signal;

performing subtraction periodically on the digital signal before band-stop filtering and the digital signal after band-stop filtering are subtracted periodically at preset time intervals to obtain a difference value;

storing the difference value in a fixed length queue, and calculating a RMS result of the data in the queue,

calculating a human impedance value based on the RMS result and a preset fitting formula, wherein, the preset fitting formula is the fitting formula obtained by fitting data based on historical RMS results and historical human impedance values; and

determining a connection state of corresponding one of the collection electrodes based on the calculated human impedance value and preset impedance measurement standards, thereby realizing abnormal monitoring of bioelectric signals attributable to abnormal connection states of the collection electrodes based on periodic determining of the connection states of the collection electrodes.

The method and device for monitoring abnormal bioelectric signals of the present application, on an aspect, based on the device of the method, especially a volume of a circuit board will not increase, which can effectively avoid a bulky shell volume of the bioelectrogram acquisition device, on the other aspect, based on the method of passive detachment monitoring, which can effectively avoid interference to an acquisition circuit.

Additional advantages, purposes, and features of the present application will be partially explained in the following description, and will become apparent to those ordinary skilled in the art after studying the following text, or can be learned according to a practice of the utility model. The purpose and other advantages of the present application can be achieved and obtained through the specific structure indicated in the description and accompanying drawings. The invention is defined in the independent claims.

## BRIEF DESCRIPTION OF THE DRAWINGS.

FIG. 1 is a flowchart of a method for monitoring abnormal bioelectric signals according to an embodiment of the present application.
FIG. 2 is a schematic diagram of an ECG detection system.
FIG. 3 is a schematic diagram of an abnormal monitoring circuit for bioelectric signals in the prior art.
FIG. 4 is a schematic diagram of a multi-electrode bioelectrogram acquisition device circuit according to an embodiment of the present application.

## DETAILED DESCRIPTION

In order to make a purpose, a technical solution, and an advantage of the present application more clearly, the following will be further described in detail below with reference to accompanying drawings and embodiments. Herein, schematic embodiments and explanations of the present application are only used to explain the present application and are not intended to limit it.

It should be emphasized that the term "including/containing" when used in the article refers to an existence of features, elements, steps, or components, but does not exclude the existence or attachment of one or more other features, elements, steps, or components.

Herein, it should also be noted that, if there are no special instructions, the term "connection" in the article can not only refer to a direct connection, but also to an indirect connection with an intermediate object.

In the following text, the embodiments of the present application will be described with reference to the accompanying drawings. In the accompanying drawings, the same reference signs represent the same or similar components, or the same or similar steps.

In order to overcome a limitation of an existing method for monitoring an abnormal bioelectric signal in a multi-electrode bioelectrogram acquisition device, the present application provides a method and device for monitoring abnormal bioelectric signals.

Electro X gram (EXG) includes electroencephalography (EEG), electrocardiography (ECG), and electromyogram (EMG). Collecting bioelectrograms is a detection method for studying potential and polarity changes that occur in organs, tissues, and cells during biological activities. A circuit system of the bioelectrogram acquisition device is a same, but a measurement accuracy of each signal is slightly different, often, a plurality of electrodes need to be connected to organisms, the bioelectrogram acquisition device includes a reference electrode (REF/ref), a right leg drive electrode (RLD), and a plurality of collection electrodes (also directly referred to as electrodes), the collection electrode serves as a positive electrode of the multi-electrode bioelectrogram acquisition device, and the reference electrode serves as a negative electrode of the multi-electrode bioelectrogram acquisition device, configured as a reference for measuring the potential of the collection electrode, a right leg drive circuit corresponding to the right leg drive electrode is usually configured for biological signal amplification to reduce common-mode interference and eliminate interference noise (mainly for household power supply noise at 50-60Hz). In a detection process of bioelectrogram, if electrode detachment is not detected in a timely manner, it may lead to data contamination.

In essence, the method for monitoring abnormal bioelectric signals proposed by the present application is based on circuit design and detection of an electrode connection state of the multi-electrode bioelectrogram acquisition device to achieve.

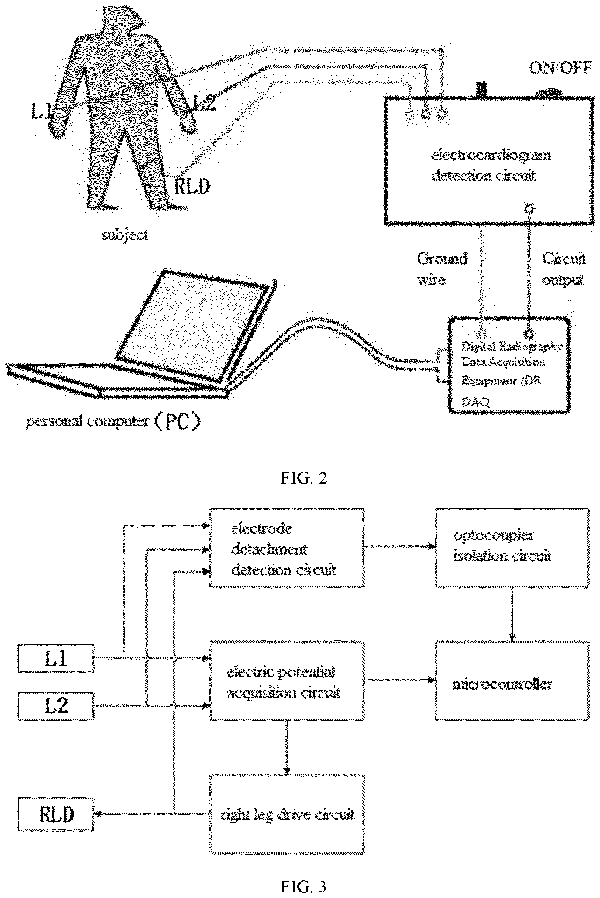

Taking an electrocardiogram (ECG) acquisition device as an example, FIG. 2 is a schematic diagram of the ECG detection system, collection electrode L1 and collection electrode L2 (In a case of electroencephalogram(EEG) acquisition device, there would be more electrodes) are connected to both hands of a subject, and the right leg drive electrode RLD is connected to the right leg of the subject to weaken common-mode interference and improve common-mode rejection ratio, a connection position of the reference electrode is generally in a weak signal area, such as an ear for EEG, and in a muscle-free area like a wrist for electromyography (EMG). All electrodes are connected to the ECG detection circuit, which is connected to a multifunctional recorder (Digital Radiography Data Acquisition Equipment, DR DAQ), to jointly detect potential changes between collection electrode L1 and the collection electrode L2, and the multifunctional recorder is connected to a personal computer to display the ECG collected by the ECG acquisition device.

Comparing with existing methods for monitoring abnormal bioelectric signals, FIG. 3 illustrates a schematic circuit diagram of an abnormal monitoring circuit for bioelectric signals in the prior art, each electrode is additionally connected to a separate abnormal monitoring circuit for bioelectric signals, which is then connected to an optocoupler-isolation circuit, and the optocoupler-isolation circuit is connected to a microcontroller to achieve abnormal bioelectric signals monitoring. It is manageable when there are only a few electrodes to collect, but if a number of electrodes increases, such as in EEG acquisition device, it will greatly increase a burden of circuit design, resulting in bulky circuit boards and even an entire device.

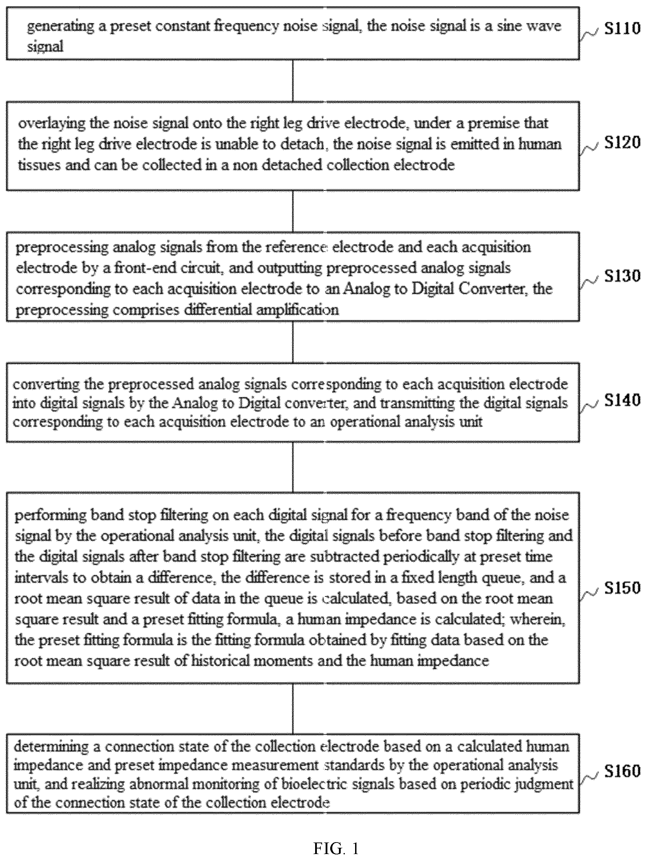

On one hand, the present application provides a method for monitoring abnormal bioelectric signals, FIG. 1 illustrates a flowchart of the method for monitoring abnormal bioelectric signals according to an embodiment of the present application, which includes the following steps:

Step S110: generate a preset constant frequency noise signal, and the noise signal is a sine wave signal.

Wherein, the noise signal in step S110 can be generated using self-excited oscillation of a Wen bridge or through a Direct Digital Frequency Synthesis (DDS). The aforementioned methods for generating noise signals are merely examples, and the present application is not limited to these methods.

Wherein, a frequency band of the noise signal in step S110 is not within the frequency band of a measurement signal, for example, an EEG frequency band is between 0.5Hz and 50Hz, It is best to keep a constant noise frequency band away from a measurement signal frequency band, in an actual development of products based on this method, a frequency of the noise signal is preset to 86Hz. Subsequently,, in step S150, a 85Hz~87Hz frequency band is filtered out through band-stop filter to eliminate interference.

Step S120: superimposingsuperimposes the noise signal onto the right leg drive electrode, under a premise that the right leg drive electrode does not detach, the noise signal is dissipated in human tissues and collected by the non-detached collection electrodes.

The noise signal of a sine wave type avoids a collection signal frequency band of the bioelectrogram acquisition device, when the connection state of the collection electrode is normal, which will be filtered out by the band-stop filtering in step S150, however, when the connection state of the collection electrode is abnormal, it is equivalent to a disappearance of the positive electrode of the collection electrode in the circuit, at this time, the positive electrode of an operational amplifier in a differential amplifier circuit is unloaded, at this time, due to the right leg drive RLD releasing a sine wave signal, an energy of a measured noise signal becomes very large, that is, an impedance becomes very large (the impedance is related to the energy of the noise signal), thus realizing a passive monitoring of abnormal bioelectric signals in the present application (i.e., abnormal electrode connection state). A function of differential amplifier circuit is to amplify an original small signal for easier acquisition by the ADC chip, the differential amplifier circuit consists of chips based on operational amplifiers.

Step S130: a front-end circuit preprocesses analog signals from the reference electrode and each collection electrode, and outputs the preprocessed analog signals corresponding to each collection electrode to an analog-to-digital converter (ADC), and the preprocessing includes differential amplification.

Wherein, the ADC chip is configured to convert analog signals into digital signals. Wherein, the front-end circuit in step S130 includes a differential amplifier circuit, a follower circuit, and a wave filter. The analog signals from the reference electrode and each collection electrode are preprocessed through the differential amplifier circuit, the follower circuit, and the wave filter.

Step S140: the ADC converts the preprocessed analog signals corresponding to each collection electrode into digital signals, and transmits the digital signals corresponding to each collection electrode to an computation and analysis unit.

Step S150: the computation and analysis unit performs band-stop filtering on each digital signal for the frequency band of the noise signal, and the digital signals before band-stop filtering and the digital signals after band-stop filtering are subtracted periodically at preset time intervals to obtain a difference, the difference is stored in a fixed length queue. A root mean square (RMS) result of the data in the queue is calculated, and a human impedance is calculated based on the RMS result and a preset fitting formula. Wherein the preset fitting formula is obtained by fitting data based on the historical RMS results and human impedance.

Based on band-stop filtering process, when the electrode connection state of the collection electrodes is normal, noise signals will not interfere with results of the bioelectrogram acquisition device. For example, for a 80Hz signal generated by a signal generator, which is processed through band-stop filtering in the frequency band of 79Hz~81Hz, and for a 86Hz signal, which is processed through band-stop filtering in the frequency band of 85Hz~87Hz.

In one embodiment of the present application, a period in step S150 can be 1-2 seconds, and a specific interval depending onan actual application scenario.

Wherein, a fixed length queue in step S150 is configured to store the difference between the digital signals before and after band-stop filtering over a period of time, the RMS result of the data obtained from the queue represents an energy of the sine wave, which corresponds to a connection quality of the electrode, that is, a magnitude of an impedance value, the energy of the sine wave is converted into the impedance value through fitting.

In some embodiments of the present application, the fitting formula is:

$$y = a_0 + a_1 x + a_2 x^2 + a_3 x^3 + \ldots + a_n x^n ;$$

wherein, a is a coefficient of each term, x is a plurality of RMS results, a plurality of coefficients a need to be fitted, and n is a preset value representing a number of terms in the fitting formula.

Step S160: the computation and analysis unit determines a connection state of the collection electrode based on a calculated human impedance and preset impedance measurement standards, and abnormal monitoring of bioelectric signals is achieved based on periodic judgment of the connection state of the collection electrode.

Wherein, types of the computation and analysis unit in step S150 and S160 include a microcontroller unit (MCU), a central processing unit (CPU), and a personal computer (PC). The present application is not limited to these, and any other hardware that meets the requirements and can complete the aforementioned computing tasks is also applicable.

In one embodiment of the present application, in step S160, the computation and analysis unit determines the connection state of the collection electrode according to the calculated human impedance and the preset impedance measurement standards, which includes: 1) if a magnitude of the human impedance does not exceed a first standard of the preset impedance measurement standard, the electrode is judged to be in a stable connection state; 2) if the magnitude of the human impedance exceeds the first standard but does not exceed a second standard of the preset impedance measurement standards, the electrode is judged to be in an unstable connection state; 3) if the magnitude of the human impedance exceeds the second standard, the electrode is judged to be in a detached state. For example, when the human impedance is within 1000 Ω, the electrode is judged to be in a stable connection state, when the human impedance is greater than 1000 Ω but not exceeding 7000 Ω, the electrode is judged to be in an unstable connection state, when the human impedance is greater than 7000 Ω, the electrode is judged to be in the detached state.

Furthermore, in another embodiment of the present application, for a case where the connection state of the collection electrode is the unstable connection state or the detached state, are determined that the electrode connection state is abnormal. Optionally, alerting for abnormal bioelectric signals can be provided on a human-computer interaction interface GUI.

Furthermore, after step S160, the method further includes: generating alert information for the collection electrode in a detected unstable connection state. For example, an alert message pops up on a bioelectrogram display interface, or a serial number of the collection electrode with detachment/unstable connection state is displayed on the bioelectrogram display interface. Alternatively, based on the detected unstable connection state of the collection electrode, the abnormal bioelectric signals is determined and an alert information is generated.

In a specific embodiment of the present application, the noise signal is overlaid on the reference electrode, when a sine wave noise signal is added to the reference electrode, if the impedance increases, this situation can still be recognized, however, if the electrode falls off, it cannot be well recognized because the sine wave signal cannot form a loop at this time, resulting in a measured noise value of almost 0, however, in reality, with good connection and impedance, the noise value is also close to 0. Although this limitation exists, it also has certain application prospects in certain specific scenarios.

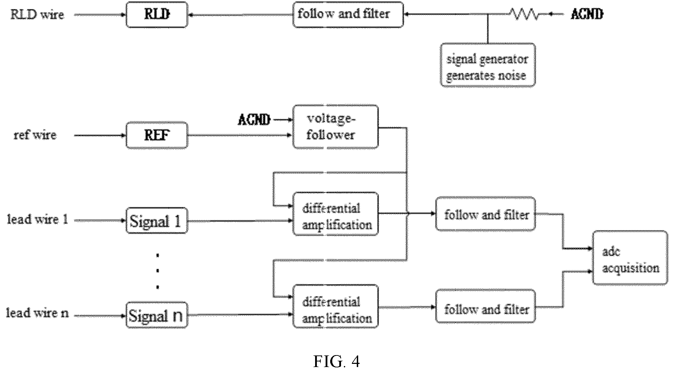

FIG. 4 shows a circuit schematic diagram of a multi-electrode bioelectrogram acquisition device according to an embodiment of the present application. Lead wires 1 to n are respectively connected to collection electrodes 1 to n, and the reference electrodes REF and are connected to REF, the signals 1 to n from reference electrodes 1 to n pass through the differential amplifier circuit, the follower circuit, and the wave filter to reach the ADC chip, the ADC chip converts the analog signal into the digital signal and sends it to the microprocessor MCU, the right leg drive electrode is connected to a RLD line, and the noise signal from the signal generator is overlaid on the right leg drive electrode and collected by the collection electrodes 1 to n through an emission in a human body. Under the premise that the connection state of the right leg drive electrode is good. When the connection state of the collection electrode in the system becomes unstable, the monitoring method provided by the present application will detect an anomaly in human impedance at the microprocessor chip MCU, thereby, thereby, determining abnormal bioelectric signals. The standard for determining abnormal human impedance is set based on an actual application scenarios of this technology. Wherein, AGND indicates a virtual ground, indicating a connection to an analog ground wire, which is mainly used in analog circuits.

A principle of the present application is based on a virtual short and virtual break principle of the operational amplifiers. The right leg drive is used to weaken common-mode noise, that is, the noise on the negative and positive electrodes of the differential amplifier circuit, the noise on both the negative and positive electrodes is reduced, and the noise obtained by the difference between the negative and positive electrodes will also be weakened. When a positive electrode detaches, there is only one negative electrode left, according to the principle of the virtual short and virtual break of the operational amplifier, at this time, there are only common-mode signals and no differential mode signals, which is equivalent to the common-mode replacing the differential mode, therefore, a special frequency band in the differential mode will be amplified to infinity. Wherein, differential amplification calculates an original signal from the collection electrodes, and this difference is a result of the filtered values before and after, which is calculated once per second.

Correspondingly to the aforementioned method, the present application further provides a device for monitoring abnormal bioelectric signals, the multi-electrode bioelectrogram acquisition device includes a reference electrode, a right leg drive electrode, and a plurality of collection electrodes, the collection electrode serves as a positive electrode of the multi-electrode bioelectrogram acquisition device, and the reference electrode serves as a negative electrode of the multi-electrode bioelectrogram acquisition device, this device includes:

1) A signal generator generates a preset constant frequency noise signal, and the noise signal is a sine wave signal.
The signal generator is a Wen bridge or a Direct Digital Frequency Synthesis DDS device, and the noise signal is generated by self-excited oscillation of the Wen bridge or by the Direct Digital Frequency Synthesis DDS. The aforementioned signal generator is merely an example, and the present application is not limited thereto. Any signal generator that is easily thought of by those skilled in the art belongs to the technical scope of protection requested by the present application.
2) The right leg drive electrode is configured to superimpose the noise signal. Under a premise that the right leg drive electrode does not detach, the noise signal is dissipated in human tissues and can be collected by the non-detached collection electrodes.
3) A front-end circuit is configured to preprocess analog signals from the reference electrode and each collection electrode, and output preprocessed analog signals corresponding to each collection electrode to an analog-to-digital converter. The preprocessing includes differential amplification.
The front-end circuit includes a differential amplifier circuit, a follower circuit, and a wave filter, the analog signals from the reference electrode and each collection electrode are preprocessed through the differential amplifier circuit, the follower circuit, and the wave filter.
4) The analog-to-digital converter is configured to convert the preprocessed analog signals corresponding to each collection electrode into a digital signal, and transmit the digital signal corresponding to each collection electrode to an computation and analysis unit.
5) The computation and analysis unit that performs band-stop filtering on each digital signal for a frequency band of the noise signal, the digital signal before band-stop filtering and the digital signal after band-stop filtering are subtracted periodically at preset time intervals to obtain a difference. The difference is stored in a fixed length queue. ARMS result of the data in the queue is calculated. Based on the RMS result and a preset fitting formula, a human impedance is calculated; The preset fitting formula is obtained by fitting data based on the RMS result of historical moments and the human impedance. The operational analysis unit also judges a connection status of the collection electrode based on the calculated human impedance and preset impedance measurement standards, and realizes abnormal monitoring of bioelectric signals based on periodic judgment of the connection status of the collection electrode.

Wherein, types of the computation and analysis unit include a microcontroller unit MCU, a central processing unit CPU, and a personal computer PC.

The method and device for monitoring abnormal bioelectric signals provided by the present application, on one hand, it is unnecessary to add a large number of bioelectric signal abnormal monitoring circuits in a multi-electrode scenario, thus, avoiding an increase in a size of a circuit board and can effectively avoid a bulky shell volume of the bioelectric signal acquisition device, on the other hand, based on a passive bioelectric signal abnormal monitoring method, it can effectively avoid interference with an acquisition circuit.

Wherein, active monitoring is a monitoring method triggered by the abnormal monitoring circuit of bioelectric signals, which can interfere with the acquisition circuit and affect an accuracy of bioelectrogram acquisition, however, the method of the present application has no action initiated by the circuit, only an additional step of band-stop filtering is added in an operational unit, a passive monitoring method provided by the present application can effectively avoid interference to the acquisition circuit, and a principle of noise signal target acquisition frequency band is generated and will be processed by band-stop filtering, so it will not affect an accuracy of bioelectrogram acquisition.

Especially for EEG acquisition scenarios, EEG acquisition is a typical scenario with multiple electrodes, if existing technology is used, there should be a corresponding monitoring circuit for each electrode, if it is 100 lead wires, one hundred monitoring circuits need to be added to the circuit board, which will increase the board volume and the shell volume, making a product bulky and very unfavorable for testing and sales.

Those skilled in the art should understand that the exemplary components, systems, and methods described in conjunction with the disclosed embodiments can be executed in the hardware, software, or a combination of both. Whether to execute it in the hardware or software depends on a specific application and design constraints of the technical solution. Professional and technical personnel can use different methods to achieve the described functions for each specific application, but such implementation should not be considered beyond the scope of the present application. When executed in the hardware, it can be, for example, electronic circuits, application specific integrated circuits (ASICs), appropriate firmware, plugins, function cards, etc. When executed in software, the elements of the present application are programs or code segments configured to execute the required tasks. The programs or the code segments can be stored in the machine readable media, or transmitted on transmission medium or communication links through the data signals carried by carriers.

It should be understood that the present application is not limited to the specific configurations and processes described above and shown in the figures. For simplicity, detailed descriptions of known methods have been omitted herein. In the above embodiments, several specific steps are described and shown as examples. However, the method process of the present application is not limited to the specific steps described and shown. Those skilled in the art can make various changes, modifications, additions, or change an order between the steps.

In the present application, the features described and/or exemplified for the embodiment can be used in the same or similar manner in one or more other embodiments, and/or combined or replaced with the features of other embodiments.

## Claims

1. A method for monitoring abnormal bioelectric signals, wherein a biological electrical signal collection device comprises a reference electrode (REF), a right leg drive electrode (RLD), and multiple collection electrodes (L1, L2, ..., Ln, wherein n is an integer greater than 2), each of the collection electrodes (L1, L2, ..., Ln) serves as a positive electrode of a multi-electrode bioelectrogram acquisition device, and the reference electrode (REF) serves as a negative electrode of the multi-electrode bioelectrogram acquisition device, **characterized in that** the method comprises the following steps:

   generating a preset constant frequency noise signal, wherein the noise signal is a sine wave signal;
   superimposing the noise signal onto the right leg drive electrode (RLD);
   preprocessing analog signals from the reference electrode (REF) and each of the collection electrodes (L1, L2, ..., Ln) by a front-end circuit, and outputting the preprocessed analog signals respectively corresponding to the collection electrodes (L1, L2, ..., Ln) to an analog-to-digital converter, the preprocessing comprises differential amplification;
   converting the preprocessed analog signals respectively corresponding to the collection electrodes (L1, L2, ..., Ln) into digital signals by the analog-to-digital converter, and transmitting the digital signals to a computation and analysis unit;
   performing following operations by the computation and analysis unit:

      performing band-stop filtering on each of the digital signals for a frequency band of the noise signal;
      performing subtraction periodically on the digital signal before band-stop filtering and the digital signal after band-stop filtering at a preset time interval to obtain a difference value;
      storing the difference value in a fixed length queue, and calculating a root mean square (RMS) result of data in the queue;
      calculating a human impedance value based on the RMS result and a preset fitting formula, wherein, the preset fitting formula is obtained by data fitting based on historical RMS results and historical human impedance values; and

determining a connection state of corresponding one of the collection electrodes (L1, L2, ..., Ln) based on the calculated human impedance value and preset impedance measurement standards, thereby realizing abnormal monitoring of bioelectric signals attributable to abnormal connection states of the collection electrodes (L1, L2, ..., Ln) based on periodic determining of the connection states of the collection electrodes (L1, L2, ..., Ln).

2. The method according to claim 1, **characterized in that**, the noise signal is generated using self-excited oscillation of a Wen bridge or produced through a Direct Digital Frequency Synthesis (DDS).

3. The method according to claim 1, **characterized in that**, the front-end circuit comprises a differential amplifier circuit, a follower circuit, and a wave filter, the analog signals from the reference electrode (REF) and each of the collection electrodes (L1, L2, ..., Ln) are preprocessed through the differential amplifier circuit, the follower circuit, and the wave filter.

4. The method according to claim 1, **characterized in that**, types of the computation and analysis unit comprise a microcontroller unit (MCU), a central processing unit (CPU), and a personal computer (PC).

5. The method according to claim 1, **characterized in that**, the determining the connection state of the corresponding one of the collection electrodes (L1, L2, ..., Ln) according to the calculated human impedance value and the preset impedance measurement standards, comprising:

    when the human impedance value does not exceed a first standard of the preset impedance measurement standards, the collection electrode is determined to be in a stable connection state;
    when the human impedance value exceeds the first standard but does not exceed a second standard of the preset impedance measurement standard, the collection electrode is determined to be in an unstable connection state;
    when the human impedance value exceeds the second standard, the collection electrode is determined to be in a detached state.

6. The method according to claim 5, **characterized in that**, the method further comprises: generating an alert information for one of the collection electrodes (L1, L2, ..., Ln) in a detected unstable connection state.

7. A device for monitoring abnormal bioelectric signals, the multi-electrode bioelectrogram acquisition device comprises a reference electrode (REF), a right leg drive electrode (RLD), and a plurality of collection electrodes (L1, L2, ..., Ln, wherein n is an integer greater than 2), each of the collection electrodes (L1, L2, ..., Ln) serves as a positive electrode of the multi-electrode bioelectrogram acquisition device, and the reference electrode (REF) serves as a negative electrode of the multi-electrode bioelectrogram acquisition device, the device comprises:

    a signal generator configured to generate a preset constant frequency noise signal, wherein the noise signal is a sine wave signal;
    the right leg drive electrode (RLD) configured to superimpose the noise signal;
    a front-end circuit configured to preprocess analog signals from the reference electrode (REF) and each of the collection electrodes (L1, L2, ..., Ln), and output the preprocessed analog signals respectively corresponding to the collection electrodes (L1, L2, ..., Ln) to an analog-to-digital converter, the preprocessing comprises differential amplification;
    the analog-to-digital converter configured to convert the preprocessed analog signals respectively corresponding to the collection electrodes (L1, L2, ..., Ln) into digital signals, and transmit the digital signals to a computation and analysis unit; and
    the computation and analysis unit configured to:

        perform band-stop filtering on each of the digital signals for a frequency band of the noise signal,
        performing subtraction periodically on the digital signal before band-stop filtering and the digital signal after band-stop filtering at a preset time interval to obtain a difference value,
        storing the difference value in a fixed length queue, and calculating a root mean square (RMS) result of the data in the queue,
        calculating a human impedance value based on the RMS result and a preset fitting formula, wherein, the preset fitting formula is the fitting formula obtained by fitting data based on historical RMS results and historical human impedance values; and
        determining a connection state of corresponding one of the collection electrodes (L1, L2, ..., Ln) based on the calculated human impedance value and preset impedance measurement standards, thereby realizing abnormal monitoring of bioelectric signals attributable to abnormal connection states of the collection electrodes (L1, L2, ..., Ln) based on periodic determining of the connection states of the collection electrodes (L1, L2, ..., Ln).

8. The device according to claim 7, **characterized in that**, the signal generator is a Wen bridge or a Direct Digital Frequency Synthesis (DDS), and the noise signal is generated by self-excited oscillation of the Wen bridge or by the Direct Digital Frequency Synthesis (DDS).

9. The device according to claim 7, **characterized in that**, the front-end circuit comprises a differential amplifier circuit, a follower circuit, and a wave filter, the analog signals from the reference electrode (REF) and each of the collection electrodes (L1, L2, ..., Ln) are preprocessed through the differential amplifier circuit, the follower circuit, and the wave filter.

10. The device according to claim 7, **characterized in that**, types of the computation and analysis unit comprise a microcontroller unit (MCU), a central processing unit (CPU), and a personal computer (PC).

**Patentansprüche**

1. Verfahren zum Überwachen abnormaler bioelektrischer Signale, wobei eine biologische elektrische Signalerfassungsvorrichtung eine Referenzelektrode (REF), eine Treiberelektrode für das rechte Bein (RLD) und mehrere Sammelelektroden (L1, L2, ..., Ln, wobei n eine ganze Zahl größer als 2 ist) umfasst, wobei jede der Sammelelektroden (L1, L2, ..., Ln) als positive Elektrode einer Multielektroden-Bioelektrogramm-Erfassungsvorrichtung dient, und wobei die Referenzelektrode (REF) als negative Elektrode einer Multielektroden-Erfassungsvorrichtung dient, **dadurch gekennzeichnet, dass** das Verfahren die folgenden Schritte umfasst:

Erzeugen eines voreingestellten Rauschsignals mit konstanter Frequenz, wobei das Rauschsignal ein Sinuswellensignal ist;
Überlagern des Rauschsignals mit der Treiberelektrode des rechten Beins (RLD);
Vorverarbeiten analoger Signale von der Referenzelektrode (REF) und allen Sammelelektroden (L1, L2, ..., Ln) durch eine Vorstufenschaltung und Ausgeben der vorverarbeiteten analogen Signale, die jeweils den Sammelelektroden (L1, L2, ..., Ln) entsprechen, an einen Analog-Digital-Wandler, wobei die Vorverarbeitung eine Differenzverstärkung umfasst;

Umwandeln der vorverarbeiteten analogen Signale, die jeweils den Sammelelektroden (L1, L2, ..., Ln) entsprechen, in digitale Signale durch den Analog-Digital-Wandler, und Übertragen der digitalen Signale an eine Berechnungs- und Analyseeinheit;
Durchführen der folgenden Operationen durch die Berechnungs- und Analyseeinheit:

Durchführen einer Bandsperrfilterung bei jedem der digitalen Signale für ein Frequenzband des Rauschsignals;
periodische Durchführung einer Subtraktion des digitalen Signals vor der Bandsperrfilterung und des digitalen Signals nach der Bandsperrfilterung in einem vorgegebenen Zeitintervall, um einen Differenzwert zu erhalten;
Speichern des Differenzwerts in einer Warteschlange mit fester Länge und Berechnen eines Effektivwerts (RMS) der Daten in der Warteschlange;
Berechnen eines Humanimpedanzwertes auf der Grundlage des RMS-Ergebnisses und einer voreingestellten Anpassungsformel, wobei die voreingestellte Anpassungsformel durch Datenanpassung auf der Grundlage von historischen RMS-Ergebnissen und historischen Humanimpedanzwerten erhalten wird; und
Bestimmen eines Verbindungszustands einer entsprechenden der Sammelelektroden (L1, L2, ..., Ln) auf der Grundlage des berechneten Humanimpedanzwerts und voreingestellter Impedanzmessstandards, wodurch eine abnormale Überwachung bioelektrischer Signale, die abnormalen Verbindungszuständen der Sammelelektroden (L1, L2, ..., Ln) zuzuschreiben sind, auf der Grundlage einer periodischen Bestimmung der Verbindungszustände der Sammelelektroden (L1, L2, ..., Ln) realisiert wird.

2. Verfahren nach Anspruch 1, **dadurch gekennzeichnet, dass** das Rauschsignal durch selbsterregte Schwingung einer Wen-Brücke oder durch eine direkte digitale Frequenzsynthese (DDS) erzeugt wird.

3. Verfahren nach Anspruch 1, **dadurch gekennzeichnet, dass** die Vorstufenschaltung eine Differenzverstärkerschaltung, eine Folgeschaltung und ein Wellenfilter umfasst, wobei die analogen Signale von der Referenzelektrode (REF) und allen Sammelelektroden (L1, L2, ..., Ln) durch die Differenzverstärkerschaltung, die Folgeschaltung und das Wellenfilter vorverarbeitet werden.

**4.** Verfahren nach Anspruch 1, **dadurch gekennzeichnet, dass** die Arten der Rechen- und Analyseeinheit eine Mikrocontrollereinheit (MCU), eine Zentraleinheit (CPU) und einen Personal Computer (PC) umfassen.

**5.** Verfahren nach Anspruch 1, **dadurch gekennzeichnet, dass** das Bestimmen des Verbindungszustandes der entsprechenden der Sammelelektroden (L1, L2, ..., Ln) gemäß dem berechneten Humanimpedanzwert und den voreingestellten Impedanzmessstandards umfasst:

wenn der Humanimpedanzwert einen ersten Standard der voreingestellten Impedanzmessstandards nicht überschreitet, wird die Sammelelektrode als in einem stabilen Verbindungszustand befindlich bestimmt;
wenn der Humanimpedanzwert den ersten Standard überschreitet, aber einen zweiten Standard des voreingestellten Impedanzmessstandards nicht überschreitet, wird die Sammelelektrode als in einem instabilen Verbindungszustand befindlich bestimmt;
wenn der Wert der Humanimpedanzwert den zweiten Standard überschreitet, wird festgestellt, dass sich die Sammelelektrode in einem gelösten Zustand befindet.

**6.** Verfahren nach Anspruch 5, **dadurch gekennzeichnet, dass** das Verfahren ferner umfasst: Erzeugen einer Warninformation für eine der Sammelelektroden (L1, L2, ..., Ln) in einem erkannten instabilen Verbindungszustand.

**7.** Vorrichtung zum Überwachen abnormaler bioelektrischer Signale, wobei die Bioelektrogramm-Erfassungsvorrichtung mit mehreren Elektroden eine Referenzelektrode (REF), eine Treiberelektrode für das rechte Bein (RLD) und eine Vielzahl von Sammelelektroden (L1, L2, ..., Ln, wobei n eine ganze Zahl größer als 2 ist) umfasst, wobei jede der Sammelelektroden (L1, L2, ..., Ln) als positive Elektrode der Multielektroden-Bioelektrogramm-Erfassungsvorrichtung dient und die Referenzelektrode (REF) als negative Elektrode der Multielektroden-Bioelektrogramm-Erfassungsvorrichtung dient, wobei die Vorrichtung umfasst:

einen Signalgenerator, der so konfiguriert ist, dass er ein voreingestelltes Rauschsignal mit konstanter Frequenz erzeugt, wobei das Rauschsignal ein Sinuswellensignal ist;
die Treiberelektrode für das rechte Bein (RLD), die so konfiguriert ist, dass sie das Rauschsignal überlagert;
eine Vorstufenschaltung, die so konfiguriert ist, dass sie Analogsignale von der Referenzelektrode (REF) und allen Sammelelektroden (L1, L2, ..., Ln) vorverarbeitet und die vorverarbeiteten Analogsignale, die jeweils den Sammelelektroden (L1, L2, ..., Ln) entsprechen, an einen Analog-Digital-Wandler ausgibt, wobei die Vorverarbeitung eine Differenzverstärkung umfasst;
den Analog-Digital-Wandler, der so konfiguriert ist, dass er die vorverarbeiteten analogen Signale, die jeweils den Sammelelektroden (L1, L2, ..., Ln) entsprechen, in digitale Signale umwandelt und die digitalen Signale an eine Berechnungs- und Analyseeinheit überträgt; und

wobei die Berechnungs- und Analyseeinheit dazu konfiguriert ist:

Durchführen einer Bandsperrfilterung an jedem der digitalen Signale für ein Frequenzband des Rauschsignals,
periodisches Durchführen einer Subtraktion des digitalen Signals vor der Bandsperrfilterung und des digitalen Signals nach der Bandsperrfilterung in einem voreingestellten Zeitintervall, um einen Differenzwert zu erhalten,
Speichern des Differenzwerts in einer Warteschlange fester Länge und Berechnen eines Effektivwerts (RMS) der Daten in der Warteschlange,
Berechnen eines Humanimpedanzwertes auf der Grundlage des RMS-Ergebnisses und einer voreingestellten Anpassungsformel, wobei die voreingestellte Anpassungsformel die Anpassungsformel ist, die durch Anpassung von Daten auf der Grundlage von historischen RMS-Ergebnissen und historischen Humanimpedanzwerten erhalten wird; und
Bestimmen eines Verbindungszustands einer entsprechenden der Sammelelektroden (L1, L2, ..., Ln) auf der Grundlage des berechneten Humanimpedanzwerts und voreingestellter Impedanzmessstandards, wodurch eine anormale Überwachung bioelektrischer Signale, die anormalen Verbindungszuständen der Sammelelektroden (L1, L2, ..., Ln) zuzuschreiben sind, auf der Grundlage einer periodischen Bestimmung der Verbindungszustände der Sammelelektroden (L1, L2, ..., Ln) realisiert wird.

**8.** Vorrichtung nach Anspruch 7, **dadurch gekennzeichnet, dass** der Signalgenerator eine Wen-Brücke oder eine Direkte Digitale Frequenzsynthese (DDS) ist, und dass das Rauschsignal durch selbsterregte Schwingung der Wen-Brücke oder durch die Direkte Digitale Frequenzsynthese (DDS) erzeugt wird.

**9.** Vorrichtung nach Anspruch 7, **dadurch gekenn-**

**zeichnet, dass** die Vorstufenschaltung eine Differenzverstärkerschaltung, eine Folgeschaltung und ein Wellenfilter umfasst, wobei die analogen Signale von der Referenzelektrode (REF) und allen Sammelelektroden (L1, L2, ..., Ln) durch die Differenzverstärkerschaltung, die Folgeschaltung und das Wellenfilter vorverarbeitet werden.

10. Vorrichtung nach Anspruch 7, **dadurch gekennzeichnet, dass** Typen der Rechen- und Analyseeinheit eine Mikrocontrollereinheit (MCU), eine zentrale Verarbeitungseinheit (CPU) und einen Personal Computer (PC) umfassen.

## Revendications

1. Procédé de surveillance de signaux bioélectriques anormaux, en ce qu'un dispositif de collecte de signaux électriques biologiques comprend une électrode de référence (REF), une électrode d'attaque de jambe droite (RLD) et de multiples électrodes collectrices (L1, L2, ..., Ln, en ce que n est un entier supérieur à 2), chacune des électrodes collectrices (L1, L2, ..., Ln) sert d'électrode positive d'un dispositif d'acquisition de bioélectrogramme à électrodes multiples, et l'électrode de référence (REF) sert d'électrode négative du dispositif d'acquisition de bioélectrogramme à électrodes multiples, **caractérisé en ce que** le procédé comprend les étapes suivantes :

la génération d'un signal de bruit de fréquence constante prédéfinie, **en ce que** le signal de bruit est un signal sinusoïdal ;
la superposition du signal de bruit sur l'électrode d'attaque de jambe droite (RLD) ;
le prétraitement de signaux analogiques à partir de l'électrode de référence (REF) et de chacune des électrodes collectrices (L1, L2, ..., Ln) par un circuit frontal, et la sortie des signaux analogiques pré-traités correspondant respectivement aux électrodes collectrices (L1, L2, ..., Ln) vers un convertisseur analogique-numérique, le prétraitement comprenant une amplification différentielle ;
la conversion des signaux analogiques pré-traités correspondant respectivement aux électrodes collectrices (L1, L2, ..., Ln) en signaux numériques par le convertisseur analogique-numérique, et la transmission des signaux numériques à une unité de calcul et d'analyse ;
l'exécution des opérations suivantes par l'unité de calcul et d'analyse :

la réalisation d'un filtrage coupe-bande sur chacun des signaux numériques pour une bande de fréquences du signal de bruit ;

la réalisation d'une soustraction périodique sur le signal numérique avant le filtrage coupe-bande et le signal numérique après le filtrage coupe-bande à un intervalle de temps prédéfini pour obtenir une valeur de différence ;
le stockage de la valeur de différence dans une file d'attente de longueur fixe, et le calcul d'un résultat de moyenne quadratique (RMS) des données dans la file d'attente ;
le calcul d'une valeur d'impédance humaine sur la base du résultat RMS et d'une formule d'ajustement prédéfinie, **en ce que**, la formule d'ajustement prédéfinie est obtenue par ajustement des données sur la base des résultats RMS historiques et des valeurs d'impédance humaines historiques ; et
la détermination d'un état de connexion de l'une des électrodes collectrices (L1, L2, ..., Ln) correspondantes sur la base de la valeur d'impédance humaine calculée et des normes de mesure d'impédance prédéfinies, réalisant ainsi une surveillance anormale de signaux bioélectriques attribuable à des états de connexion anormaux des électrodes collectrices (L1, L2, ..., Ln) sur la base de la détermination périodique des états de connexion des électrodes collectrices (L1, L2, ..., Ln).

2. Procédé selon la revendication 1, **caractérisé en ce que** le signal de bruit est généré à l'aide d'une oscillation auto-excitée d'un pont de Wien ou produit par une synthèse de fréquence numérique directe (DDS).

3. Procédé selon la revendication 1, **caractérisé en ce que** le circuit frontal comprend un circuit amplificateur différentiel, un circuit suiveur et un filtre à ondes, les signaux analogiques provenant de l'électrode de référence (REF) et de chacune des électrodes collectrices (L1, L2, ..., Ln) sont pré-traités par le circuit amplificateur différentiel, le circuit suiveur et le filtre à ondes.

4. Procédé selon la revendication 1, **caractérisé en ce que** des types de l'unité de calcul et d'analyse comprennent une unité de microcontrôleur (MCU), une unité centrale de traitement (CPU) et un ordinateur personnel (PC).

5. Procédé selon la revendication 1, **caractérisé en ce que** la détermination de l'état de connexion de l'une des électrodes collectrices (L1, L2, ..., Ln) correspondantes selon la valeur d'impédance humaine calculée et les normes de mesure d'impédance prédéfinies, comprenant :

lorsque la valeur d'impédance humaine ne dépasse pas une première norme des normes de mesure d'impédance prédéfinies, l'électrode collectrice est considérée comme étant dans un état de connexion stable ;

lorsque la valeur d'impédance humaine dépasse la première norme mais ne dépasse pas une deuxième norme de la norme de mesure d'impédance prédéfinie, l'électrode collectrice est considérée comme étant dans un état de connexion instable ;

lorsque la valeur d'impédance humaine dépasse la deuxième norme, l'électrode collectrice est considérée comme étant dans un état déconnecté.

6. Procédé selon la revendication 5, **caractérisé en ce que** le procédé comprend en outre : la génération d'une information d'alerte pour l'une des électrodes collectrices (L1, L2, ..., Ln) dans un état de connexion instable détecté.

7. Dispositif de surveillance de signaux bioélectriques anormaux, en ce que le dispositif d'acquisition de bioélectrogramme à électrodes multiples comprend une électrode de référence (REF), une électrode d'attaque de jambe droite (RLD) et une pluralité d'électrodes collectrices (L1, L2, ..., Ln, en ce que n est un entier supérieur à 2), chacune des électrodes collectrices (L1, L2, ..., Ln) sert d'électrode positive du dispositif d'acquisition de bioélectrogramme à électrodes multiples, et l'électrode de référence (REF) sert d'électrode négative du dispositif d'acquisition de bioélectrogramme à électrodes multiples, le dispositif comprend :

un générateur de signaux configuré pour générer un signal de bruit de fréquence constante prédéfinie, en ce que le signal de bruit est un signal sinusoïdal ;

l'électrode d'attaque de jambe droite (RLD) configurée pour superposer le signal de bruit ;

un circuit frontal configuré pour pré-traiter des signaux analogiques provenant de l'électrode de référence (REF) et de chacune des électrodes collectrices (L1, L2, ..., Ln), et la sortie des signaux analogiques pré-traités correspondant respectivement aux électrodes collectrices (L1, L2,..., Ln) vers un convertisseur analogique-numérique, le prétraitement comprend une amplification différentielle ;

le convertisseur analogique-numérique configuré pour convertir les signaux analogiques pré-traités correspondant respectivement aux électrodes collectrices (L1, L2, ..., Ln) en signaux numériques, et la transmission des signaux numériques à une unité de calcul et d'analyse ; et

l'unité de calcul et d'analyse configurée pour :

effectuer le filtrage coupe-bande sur chacun des signaux numériques pour une bande de fréquences du signal de bruit,

effectuer la soustraction périodique sur le signal numérique avant le filtrage coupe-bande et le signal numérique après le filtrage coupe-bande à un intervalle de temps prédéfini pour obtenir une valeur de différence,

stocker la valeur de différence dans une file d'attente de longueur fixe, et calculer un résultat de moyenne quadratique (RMS) des données dans la file d'attente,

calculer une valeur d'impédance humaine sur la base du résultat RMS et d'une formule d'ajustement prédéfinie, en ce que, la formule d'ajustement prédéfinie est la formule d'ajustement obtenue par ajustement des données sur la base des résultats RMS historiques et des valeurs d'impédance humaines historiques ; et

déterminer un état de connexion de l'une des électrodes collectrices (L1, L2, ..., Ln) correspondantes sur la base de la valeur d'impédance humaine calculée et des normes de mesure d'impédance prédéfinies, réalisant ainsi une surveillance anormale de signaux bioélectriques attribuable à des états de connexion anormaux des électrodes collectrices (L1, L2, ..., Ln) sur la base de la détermination périodique des états de connexion des électrodes collectrices (L1, L2, ..., Ln).

8. Dispositif selon la revendication 7, **caractérisé en ce que** le générateur de signaux est un pont de Wien ou une synthèse de fréquence numérique directe (DDS), et le signal de bruit est généré par une oscillation auto-excitée du pont de Wien ou par la synthèse de fréquence numérique directe (DDS).

9. Dispositif selon la revendication 7, **caractérisé en ce que** le circuit frontal comprend un circuit amplificateur différentiel, un circuit suiveur et un filtre à ondes, les signaux analogiques provenant de l'électrode de référence (REF) et de chacune des électrodes collectrices (L1, L2, ..., Ln) sont pré-traités par le circuit amplificateur différentiel, le circuit suiveur et le filtre à ondes.

10. Dispositif selon la revendication 7, **caractérisé en ce que** des types de l'unité de calcul et d'analyse comprennent une unité de microcontrôleur (MCU), une unité centrale de traitement (CPU) et un ordinateur personnel (PC).

generating a preset constant frequency noise signal, the noise signal is a sine wave signal ∿ S110

overlaying the noise signal onto the right leg drive electrode, under a premise that the right leg drive electrode is unable to detach, the noise signal is emitted in human tissues and can be collected in a non detached collection electrode ∿ S120

preprocessing analog signals from the reference electrode and each acquisition electrode by a front-end circuit, and outputting preprocessed analog signals corresponding to each acquisition electrode to an Analog to Digital Converter, the preprocessing comprises differential amplification ∿ S130

converting the preprocessed analog signals corresponding to each acquisition electrode into digital signals by the Analog to Digital converter, and transmitting the digital signals corresponding to each acquisition electrode to an operational analysis unit ∿ S140

performing band stop filtering on each digital signal for a frequency band of the noise signal by the operational analysis unit, the digital signals before band stop filtering and the digital signals after band stop filtering are subtracted periodically at preset time intervals to obtain a difference, the difference is stored in a fixed length queue, and a root mean square result of data in the queue is calculated, based on the root mean square result and a preset fitting formula, a human impedance is calculated; wherein, the preset fitting formula is the fitting formula obtained by fitting data based on the root mean square result of historical moments and the human impedance ∿ S150

determining a connection state of the collection electrode based on a calculated human impedance and preset impedance measurement standards by the operational analysis unit, and realizing abnormal monitoring of bioelectric signals based on periodic judgment of the connection state of the collection electrode ∿ S160

FIG. 1

FIG. 2

FIG. 3

FIG. 4

## REFERENCES CITED IN THE DESCRIPTION

**Patent documents cited in the description**

- CN 205215225 U
- CN 102613969 A
- JP H11332839 A